(19) Europäisches Patentamt
European Patent Office
Office européen des brevets

(11) Publication number: **0 420 435 A2**

# (12) EUROPEAN PATENT APPLICATION

(21) Application number: **90309884.6**

(51) Int. Cl.[5]: **A61F 2/32**

(22) Date of filing: **10.09.90**

(30) Priority: **28.09.89 GB 8921878**

(43) Date of publication of application:
**03.04.91 Bulletin 91/14**

(84) Designated Contracting States:
**AT BE CH DE DK FR GB IT LI NL SE**

(71) Applicant: **IMPERIAL CHEMICAL INDUSTRIES PLC**
**Imperial Chemical House, Millbank**
**London SW1P 3JF(GB)**

(72) Inventor: **Jones, Michael Edward Benet**
**45 Richmond Crescent**
**Vicars Cross, Chester(GB)**
Inventor: **Taylor, David**
**27 Carysfort Road**
**Dalkey, County Dublin(IR)**

(74) Representative: **Thomas, Ieuan et al**
**Imperial Chemical Industries PLC, Legal Department: Patents, PO Box 6, Bessemer Road**
**Welwyn Garden City, Herts, AL7 1HD(GB)**

## (54) Prosthetic devices.

(57) A prosthetic device which comprises both a modulus matched component having a safety factor of more than about 2 and a second, typically metal, component. Such prosthetic devices are particularly applicable to hip-joint replacements.

Fig 1

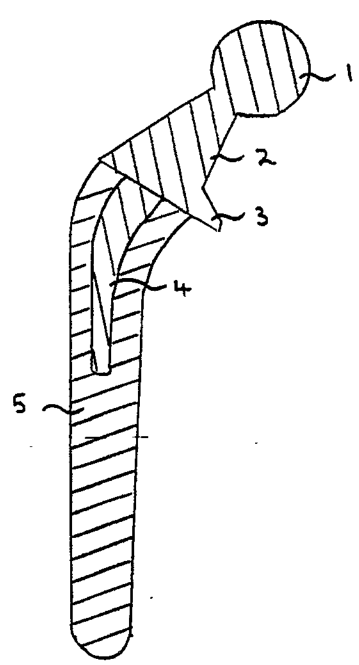


EP 0 420 435 A2

Xerox Copy Centre

## PROSTHETIC DEVICES

The present invention relates to composite materials for use in orthopaedics, to processes for preparing the composite materials, to prosthetic devices (ie shaped products adapted for location and use within the body) incorporating them, and particularly to so-called endoprostheses, ie to the artificial replacement of parts of the anatomy internally resident in the human or animal body, principally bone.

Many endoprosthetic devices are known to the skilled man in the bioengineering art. As examples of such devices may be mentioned inter alia so-called "bone plates" which are used to hold fractured bones in alignment during healing; plates or inserts used to cover damage to a bone or to replace damaged or destroyed bone or to reinforce it, for example in the skull or long bones; and components of replacement joints, particularly the hip and knee joints, although the invention is not limited to such devices. Such devices must be of a shape and configuration and have properties appropriate to their proposed application. Preferably, such that they are acceptable to the living tissue with which they are likely to come into contact when in use for a time sufficient for them to perform their intended function without unacceptable damage or undue discomfort to the recipient. More preferably, they are sufficiently acceptable for a period such that where appropriate they need not be removed from the body during the life of the recipient.

Animal bone and ivory have, in the past, been employed in orthopaedic prostheses. As with all natural materials, however, it is difficult to ensure the supply of these materials with adequate and predictable properties. Moreover, they contain protein which is not of the patient's origin and this biological incompatibility often initiates an adverse response from the body tissue with which they are in contact. For example, inflammatory reactions or rejection may arise. Accordingly, surgeons have resorted, and still resort, to a variety of synthetic engineering materials, the envisaged uses of which materials were often remote from prostheses.

For example, in total hip replacement, often the femoral head is replaced with a metal alloy, typically an austenitic stainless steel or Co-Cr alloy. The aforesaid metal alloy is secured in the femur, typically by a polymethylmethacrylate bone cement, and is seated in a high molecular weight, high density polyethylene acetabular cup. The alloys used are selected primarily by virtue of their resistance to corrosion, adequate fracture toughness, creep and fatigue strength. They have proved satisfactory but less than ideal, in such applications.

Attention has recently been devoted to the development of even more "bioinert" prosthetic materials with comparable strength properties. Examples of such bioinert prosthetic materials include titanium alloys and ceramics, principally alumina. The brittle nature of ceramics, however, presents further problems for prostheses. Furthermore, materials such as plastics and composite materials comprising for example a long fibre-reinforced polymer material, eg carbon-fibre-filled thermoplastics, have been suggested for use in prosthetic devices.

All such materials have been found to have disadvantages. For example, devices comprising fibre-reinforced composites sometimes initiate a tissue reaction. Such tissue reaction may be a response to the chemical nature of the matrix of the composite or may result from the presence of fine particles of the fibrous component of the device, resulting possibly from wear and/or abrasion of the fiber where it is present at or adjacent the surface of the device. Where carbon fiber is used as fibrous reinforcement in a prosthetic device which is disposed near certain surfaces of the human body, eg the face, the inherent black colour of such devices often leads to cosmetic problems, moreover, it has been suggested that C fiber particles may be carcinogenic. Metal and ceramic devices tend to have too high a modulus so that the adjacent bone tends to regress during extended periods of use, resulting in loosening of the device.

We have now devised prosthetic devices, particularly for hip joints, which generate stresses in bone adjacent thereto similar to stresses in healthy bone at the site of replacement. Such prosthetic devices comprise both a first component which comprises a composite and a second component. The aforesaid composite is modulus matched and comprises a cured resin and a particulate inorganic solid as hereinafter described in more detail. The aforesaid second component is formed from the metal and/or ceramic material.

The stresses in bone surrounding the above composite component are often higher than in bone surrounding components of similar shape, size and disposition in wholly metal or ceramic prosthetic devices. For example, where the prosthetic device according to the present invention is a hip-joint replacement the stresses in the upper (proximal) part of the bone are much higher and closer to those of intact femur.

Indeed, we have estimated that where the prosthetic device according to the present invention provides a hip-joint replacement the aforementioned stresses in the bone surrounding a stem formed from the

composite component approach those of cortical bone which it replaces and the stresses in the prosthetic device are reduced.

By "modulus matched" we mean a similar Young's modulus to that of cortical bone in contact with the prosthetic device, eg 7-30 GPa and typically about 20 GPa. It will be appreciated that the Young's modulus of cortical bone depends on inter alia the size, weight and age of the recipient of the prosthetic device and the site in the recipient's body in which it is to be disposed.

According to a first aspect of the present invention there is provided a prosthetic device characterised in that it comprises both a first component which comprises a modulus matched composite and which has a factor of safety (hereinafter referred to for convenience as "FOS") of more than about 2, and preferably about 3, and a second component.

Whereas the following portions of this specification describe hip, and particularly femoral replacement in detail, it will be appreciated that the invention is applicable to articulate prosthetic devices generally, particularly those in which a stem is disposed in a long bone, eg knee and elbow joints.

It will be appreciated that the prosthetic device according to the first aspect of the present invention will be chosen such that the shape, configuration and chemical properties thereof are appropriate to the proposed location and disposition thereof within the living body. Preferably the prosthetic device according to the first aspect of the present invention provides a femoral replacement.

Where the prosthetic device according to the first aspect of the present invention is a femoral prosthetic device the first component preferably provides the femoral stem and the second component provides at least the head. More preferably the head is formed with an integral neck and a projection therefrom which is disposed in the stem.

FOS is defined as the fatigue limit measured by a 3-point bending test divided by the maximum tensile service stress of the prosthetic device. Where the prosthetic device according to the first aspect of the present invention is a femoral replacement and the first component provides the stem, the so-called "maximum principal stress" would be used as the maximum tensile service stress.

According to a second aspect of the present invention there is provided a prosthetic device in the form of a femoral replacement characterised in that it comprises at least both a composite stem and a second component provided with a metallic articulate surface and wherein for an applied load of 3 kN, the stresses within the medial and lateral sides of the femoral replacement are not more than about those found in physiological material at that site. Preferably the aforementioned stresses are not more than about 50 MPa compressive and about 20 MPa tensile respectively.

According to a further aspect of the present invention there is provided:

a process for the preparation of a prosthetic device which process comprises the steps of

A Estimating the target properties in a prosthetic device of known shape and size;

B Computing the stresses therein and in the bone adjacent thereto using FEA and photoelastic stress analysis;

C Redesigning a prosthetic device from the data generated in Steps A and B to produce physiological level of stresses in the said bone;

D Choosing a composite having the target properties and a suitable FOS for that application from the stress analysis produced in Step B and mode of fatigue failure;

E Curing a curable composition in a suitable mould for the redesigned prosthetic device from Step C to produce the composite chosen in Step D;

F Testing the redesigned prosthetic device from Step E in full-scale laboratory simulations observing the time and mechanism of failure;

G Further redesigning the prosthesis from the data generated in Step F and optionally choosing a new composite to improve both FOS and stress transfer from the prosthesis to the adjacent bone; and

H Iterating the above Steps.

By FEA we mean so-called "finite element analysis".

Typically, in Step A of the process according to the further aspect of the present invention, the target properties are set such that physiological levels of stress are generated in the said bone.

Where the prosthetic device prepared by the process according to the further aspect of the present invention is a femoral replacement, the known device used in Step A is typically a so-called Sheehan metallic hip joint comprising a large medial collar and a broad proximal medial-lateral dimension.

The stresses in Steps A and B would be computed by the skilled man from the Young's modulus and certain other mechanical or elastic properties, eg Poisson's ratio, of a composite. They could be computed using an appropriate software package, typically a commercially available package, eg PAFEC, as is more fully described by Prendergast et al in Clinical Materials, 1989, 4 , 361.

Guidance for making the choice of curable composition for use in Step E is described in more detail

hereinafter.

Further parameters which are required in Step C will be known to the skilled man in the biomechanical engineering art.

Where the prosthetic device according to the first aspect of the present invention provides a femoral replacement, and in the prosthetic device according to the second aspect of the present invention, the second component provides the bearing-surface for the femoral replacement in contact with the socket. Whereas the aforesaid bearing-surface may be present as a coating on a femoral head prepared from the composite, preferably the femoral head is formed from the second component, eg a ceramic or preferably a metal. Where the head is metallic it is typically formed with a neck, collar and projection therefrom, eg a spike, which is disposed within the proximal end of the stem such that it tends to form a core within a sheath of the composite as is more fully described hereinafter with reference to Figure 1. However, alternative methods of attachment of the head to the stem will be known to, or can be readily found by, the skilled man.

The prosthetic device according to the first aspect, where it is a femoral replacement, and the prosthetic device according to the second aspect, of the present invention are preferably provided with a collar which provides direct compressive contact between the device and adjacent bone at the aforementioned proximal end of the femur and generates higher axial stresses therein. However, we do not exclude the possibility that those prosthetic devices do not comprise a collar.

We have found that the compressive stresses in the calcar region of the femur in contact with the prosthetic device according to the second aspect of the present invention bearing a collar are typically between 30 and 40 MPa when the hip joint is loaded in the normal physiological manner with a load of 3 kN. "3 kN" is generally accepted in the art as about the peak load for a 70 kg man during normal walking.

The size of the prosthetic device will be chosen in the light of inter alia the size and age of the patient in which it is to be disposed.

The relative shapes and sizes of the first and second components will be determined by the skilled man by experiment and/or calculation. The first component often provides a major portion by volume of the prosthetic device. It will be appreciated that the size/shape of the second component should not unduly reduce the stress-transferring properties of the first component.

We do not exclude the possibility that where the first component provides the stem of a prosthetic device, eg a femoral hip replacement, it may have a sheath of a composite having a lower modulus than the bulk composite, eg by using a sheath of composite which has a lower particulate inorganic filler content, to facilitate stress transfer to the bone. It may further, or alternatively, be provided with a porous surface, or means to generate a porous surface in situ, eg using HAP, to facilitate bone integration.

Where the second component is metallic it will typically be made of a metal conventionally used in prosthetic devices, for example stainless steel, a cobalt/chromium alloy or preferably a titanium alloy. It will be chosen in the light of inter alia its modulus, wear characteristics and resistance to corrosion in the body.

Where the second component is ceramic it will typically be made of a ceramic conventionally used in prosthetic devices, eg an inert oxide ceramic.

The prosthetic devices according to the first and second aspects of the present invention are typically prepared by (i) disposing the second component of appropriate shape and size in a suitable mould; (ii) charging the mould with a curable composition; and (iii) curing the composition to form the composite with a portion of second component disposed therein.

The curable composition is in the form of an intimate admixture, a dispersion or suspension (which terms are used interchangeably herein) of at least one particulate inorganic solid in a resin. It will be appreciated that the at least one particulate inorganic solid acts as a reinforcement in the composite component.

The composite of which the prosthetic device according to the present invention is comprised has low water extractables, preferably less than 0.15%; has low water uptake, preferably less than 0.3%; and can be autoclaved with no undue detrimental affect.

"Water extractibles" and "water-uptake" are defined hereinafter.

The resin when cured per se gives a product which (a) has a flexural modulus of about 3 GPa; (b) has a flexural strength and modulus which remain substantially unchanged when autoclaved in steam at about 120 °C for about 20 minutes; and (c) remains substantially unchanged when saturated with water at 74 °C.

The resin bears on average more than one addition-polymerisable olefinically unsaturated carbon-carbon double bond per molecule. It is often prefered that it comprises a plurality of such carbon-carbon double bonds. The resin, which may be neat or an admixture, will be chosen (a) in the light of (i) the processability properties required in the curable composition (ii) the properties of the prosthetic device according to the present invention and (b) such that the curable composition does not shrink unduly on

curing.

As examples of resins bearing a plurality of addition-polymerisable carbon-carbon double bonds may be mentioned inter -alia monomers, eg

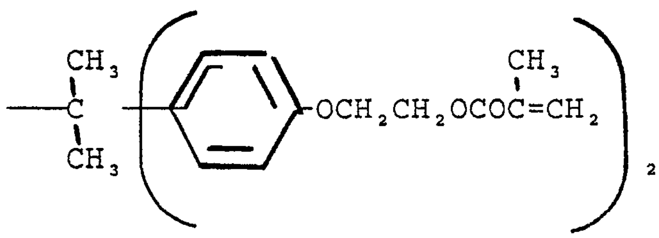

or oligomers.

As examples of such oligomers may be mentioned inter alia unsaturated aromatic-compound/aldehyde oligomers, eg as described in our European Patent Specifications Nos 0,112,650A and 0,162,651A.

As examples of addition-polymerisable monomers bearing one addition-polymerisable carbon-carbon double bond which may be used as comonomers with resins bearing a plurality of C-C double bonds may be mentioned inter alia alkyl (alk)acrylates, eg methyl methacrylate, or preferably cyclohexyl methacrylate; or 2, 3, 4, 5, 6-pentafluorostyrene.

The presence of a comonomer in the curable composition often reduces the viscosity thereof and allows certain properties, eg water-uptake; of the prosthetic device prepared therefrom to be adjusted. Where the comonomer contains one carbon-carbon double bond it reduces the cross-link density of the composite and hence tends to improve certain of the mechanical properties of the prosthetic device.

The viscosity of the resin is, or is adapted, such that sufficient of the inorganic particles may be mixed therewith such that on curing the curable composition, a composite which has the flexural modulus hereinbefore described is obtained.

The particulate inorganic solid is stiff enough to impart desired rigidity to the prosthetic device and is substantially non-leachable, ie stable for extended periods, eg more than 10 years, in a moist environment.

As examples of suitable particulate inorganic solids which may be used in curable compositions may be mentioned inter alia hydroxy-apatite, talc, mica, alumina, silica or preferably a glass, eg a borosilicate, or more preferably a radio-opaque glass, eg Raysorb T3000 (RTM).

We do not exclude the possibility that a portion, up to 10% v/v say, of the particulate inorganic solid may be in the form of, for example, plates or fibrils.

Preferably, the particulate inorganic solid is treated with a suitable coupling agent to improve the bonding thereof to the resin. For example, where the particulate inorganic solid is silica it may be treated with a suitable silane coupling agent, eg $\gamma$-methacryl-oxypropyl-trimethoxysilane.

Often a stabiliser or dispersing agent, eg a long chain amine or phosphate (as is more fully described in our European Patent Specification No 0 013 491), is used in the curable composition. Use of such an agent tends to improve the stability of the dispersion of the particulate inorganic solid in the resin and to facilitate the shaping of the curable composition in a mould.

It will be appreciated that the volume % of the particulate inorganic solid in the curable composition will depend on inter alia the size, shape and size distribution of the particles, and the efficacy of dispersion of the particles in the resin. These will be chosen such that the curable composition has sufficient mobility to allow the shaping/fabrication thereof to be carried out and the composite to have the desired modulus. The lower limit of the volume % of the particulate inorganic solid will be readily determined by the skilled man by simple experiment.

The curable composition contains sufficient particulate inorganic solid to give a composite having a Young's modulus of 7-30 GPa, ie the composite is modulus matched as hereinbefore defined. The curable composition and the composite preferably comprise 20-80%, more preferably 40-75% and particularly more preferably 65-70% v/v, it is found that the particulate inorganic solid often can not be distributed homogeneously in the resin. Below about 20% v/v particulate inorganic solid, the composite component of prosthetic devices prepared by polymerisation of the curable composition tends to be more complaint than any femur bone. The particulate inorganic solid tends both to reinforce the composite and to enhance its stiffness.

The particulate inorganic solid may be used in the form of ground particles, preferably wherein the particle size is from 90% thereof being less than 100 µm to 1.0 µm, preferably from 90% being less than 50 µm to 0.1 µm. Preferably a portion of th particles are sub-micron particles, more preferably about 10%

thereof are such particles, eg of silica. The use of such sub-micron particles tends to facilitate the obtaining of a high volume % particle content. Mixtures of particles of different inorganic solids often further facilitate the obtaining of a high volume % particle content.

The particulate inorganic solid may also be used in the form of acicular particles or platelets, the latter preferably having a maximum length of 500 μm and a maximum thickness of 20 μm.

The composites prepared by polymerisation of the curable composition have a Young's modulus in the range of values recorded for compact bone, preferably between 15 and 30 GPa, more preferably about 20 GPa, as hereinbefore mentioned.

Either or both the femoral portion, ie the head and stem, and the acetabular cup into which the head seats in vivo may comprise the composite, but use of the composite as or in the acetabular cup is not preferred. Where the prosthetic device provides the cup it will typically be provided with a wear-resistant coating.

We do not exclude the possibility that the composite may be reinforced by incorporation of a suitable fibre which is stable in the human body, eg glass, in the composite.

It will be appreciated that the prosthetic device should be biocompatible, ie the metal or ceramic of which the second component is comprised, the particulate inorganic filler, any additive, a fibre, where present, and the cured resin should be substantially non-toxic to mammals, particularly humans, and that minimal amounts, less than 0.2% say, of water extractables should be extractable therefrom on exposure to water at 74°C until saturated.

The present invention is further illustrated by reference to the following drawings which show, by way of example only, one embodiment of a device according to the present invention, certain components therefor and certain stresses associated within, and arising from, the use thereof.

In the drawings:

Figure 1 is a longitudinal section of a femoral replacement according to the present invention;

Figure 2 is a diagrammatic representation of a metallic component for use in the femoral replacement illustrated in Figure 1;

Figure 3 illustrates the predicted stresses in the femur bone, up to about 23 mm from the proximal end, as a function of distance from the proximal end at the point immediately under the collar of the femoral replacement arising from the use of a certain prosthetic device; and

Figure 4 illustrates the stresses over substantially the whole length in the stem of a femoral prosthetic device according to the present invention, as a function of the distance from the proximal end.

In Figure 1, a femoral head comprising an integral titanium ball (1), neck (2), collar (3) and spike (4) is firmly attached to stem (5).

In Figure 2, the same part numberings denote the same parts.

In Figure 3, lines (A) and (B) represent the stresses predicted in femur bone adjacent the stem of a prosthetic device according to the present invention and a cobalt/chromium prosthetic device respectively.

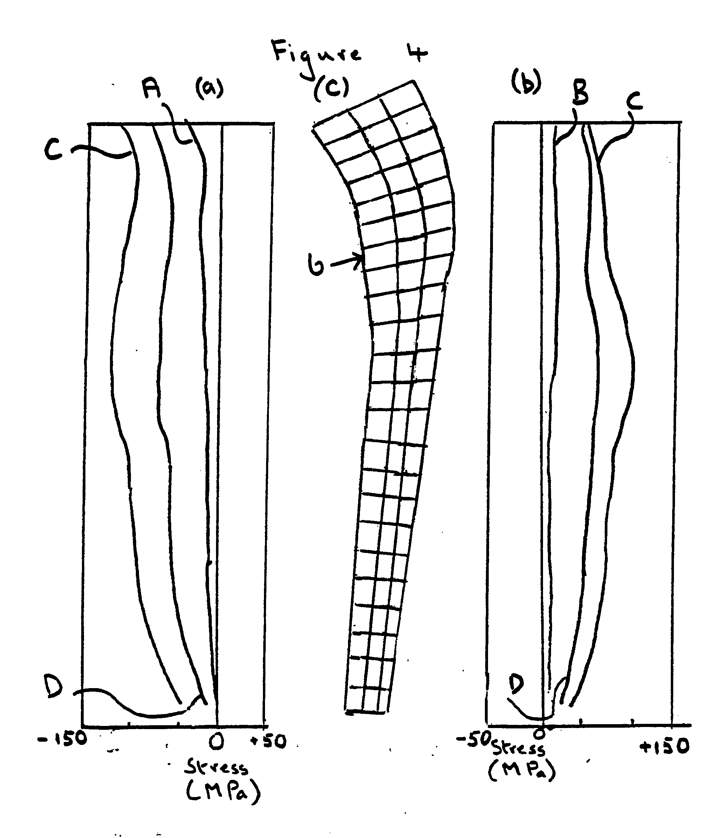

In Figure 4 (a). Line A indicates the stresses in the medial side of the prosthesis according to the present invention with increasing distance from the proximal end. In Figure 4(b), Line B indicates the stresses in the lateral side of the prosthesis according to the present invention with increasing distance from the proximal end. For comparison in Figure 4, Lines C and D indicate the stresses in prosthetic devices prepared from Cr/Co alloy and Ti alloy respectively.

In Figure 4 (a and b) the vertical axis corresponds to the positions illustrated on the prosthesis (6) in Figure 4(c).

Preparation of the curable composition and a prosthetic device according to the present invention may be effected by processes well known in the art. For example, the resin and the particulate inorganic solid may be mixed by known techniques, eg rolling, calendering, milling or Z-blade mixing. We do not exclude the possibility that the mixture of the resin and the particulate inorganic solid may be diluted with a low-boiling solvent in order to provide a curable composition of the desired fluidity; the low boiling solvent being caused or allowed to evaporate before the curable composition is cured. However, this possibility is not preferred.

Curing may be effected by techniques well known in the resin art, eg a peroxide cure using a suitable peroxide. Preferably curing is effected at an elevated temperature and pressure, eg about 80°C, using a peroxide having a suitable half-life at that temperature, eg t-butyl per-2-ethyl-hexanoate. Such a curing technique has the advantage that the viscosity of the curable composition is lowered (hence it flows more readily and at least reduces the voidage in the composite) and/or mould times are reduced and the properties of the composite are improved as a result, it is believed, of a higher degree of cure.

Where the curable composition is transparent or translucent we do not exclude the possibility that the curing thereof may be initiated by a light curing catalyst, eg as described in our UK Patent Specifications

Nos 1, 408,265 and 1.494,903 or our European Patent Specifications Nos 0,059,649 and 0,090,493. However, this is not preferred.

The present invention is further illustrated by reference to the following Examples.

Water Test

"Water extractibles" and "water uptake" were determined by the following procedure.

A moulded sheet (0.3 cm x 3 cm x 1 cm) (Weight A) of the composite was immersed in water at 74° C until it reached constant weight (typically after more than 400 hrs) (Weight B). The moulded sheet was then dried under vacuum at 40° C until it reached constant weight (Weight C). The above parameters were then calculated from the equations:

$$\text{Water uptake} = \frac{B-A}{A} \times 100\%$$

and

$$\text{Water extractibles} = \frac{A-C}{A} \times 100\%$$

Examples 1-3

These Examples illustrate composites for use in a prosthetic device according to the present invention.

General procedure

The dimethacrylate of oxyethylated bis-phenol A (Diacryl 101, RTM) (100 gms), cyclohexyl methacrylate (100 gms), p-methoxy phenol (0.13 gms) and t-butyl per-2-ethyl-hexanoate (1.0 gms) were stirred at room temperature for 20 minutes and then at a vacuum of (10160 Pa) for 30 seconds. A portion (100 gms) of that mixture, silane coupling agent (A174; 1.4 gms), n-dodecylamine (0.7 gms), and di(2-ethyl-hexyl) phosphate (0.8 gms) were stirred for 5 minutes at room temperature to give Mixture B.

Silica powder (Aerosil OX50 (RTM); 60 gms) was added to a portion (100 gms) of Mixture B and stirred until a uniform dispersion was obtained.

Powder of a barium borosilicate glass (Raysorb T3000 (RTM)), was added to the dispersion until stirring became difficult. The viscous mixture was then transferred to a mechanical Z-blender and stirring was continued with the addition of further amounts of the glass powder, until the following quantities of T3000 were present, Ex 1: 134.1 g; Ex 2: 197.1 g and Ex 3: 428.2 g. Stirring was continued for 5 minutes and then for a further 2 minutes under a vacuum of 8800 Pa to afford a white, slightly tacky semi-solid, which was found by pyrolysis to have the ash content indicated in Table 1.

A sheet (thickness 3 mm) was prepared by compression moulding a sample (70 gms) of the semi-solid under the following cycle:

80° C/5 tons gauge/30 mins; and 110° C/5 tons gauge/30 mins

using a Moore and Son (Birmingham) Ltd hand-operated press with platens measuring 20 x 20 cms.

A strong, stiff moulding was obtained and the curing thereof was completed by heating at 150° C for 30 minutes in a fanned oven. The flexural modulus and flexural strength of the product from the oven were determined.

The Young's modulus and fracture toughness ($K_{1c}$; by the method of Pilliar et al, J. Biomedical Materials Research, 1987, 21 , 145) of these products were determined.

The results are shown in Table 1.

From Table 1 it can be seen that the Young's modulus increases with the filler concentration.

Example 4

This Example illustrates the preparation of a prosthetic device according to the present invention.

Ethoxylated-bisphenol-A dimethacrylate (Diacryl 101, RTM; 50 gms), cyclohexyl methacrylate (50 gms), p-methoxyphenol (0.05 gms) t-butyl peroxy-2-ethyl hexanoate (0.5 gms), silane coupling agent (A174; 1.4 gms). $C_{12/14}$ amine dispersing agent (0.72 gms), and diethylhexylphosphate (0.8 gms) were stirred at room temperature for 10 minutes to give mixture A.

Silica powder (Aerosil OX50, RTM; 64.8 gms) was progressively added to mixture A and hand mixed until a

TABLE 1

| Example No | Filler Fraction | | Young's Modulus[a] (GPa) | Fracture Toughness ($MN^{-3/2}$) |
|---|---|---|---|---|
| | (wt%) | (volume%) | | |
| 1 | 66 | 45 | 11 | 1.1 |
| 2 | 72 | 51 | 12 | 1.1 |
| 3 | 83 | 67 | 18 | 1.45 |

a : Loading rate of 2 mm.min

uniform dispersion was obtained. Powder of a barium borosilicate glass (Raysorb T3000, RTM; 570.8 gms) was added to the dispersion in a Z-blender over about 1 hour. The mixture so obtained was then mixed for a further 4 hours and degassed for 3 minutes under 8,000 Pa, to give mixture B.

A portion (200 gms) of mixture B was charged to the transfer chamber of a transfer mould containing a stainless steel head. A simplified shape was used for the head (ie rectangular shape 1.2 x 3.2 x 6 $cm^3$, with a spike 5 cm long, 1 cm diameter extending therefrom.) Similarly a simplified shape was used for the composite stem. The prosthetic device was transfer moulded at room temperature and then cured at 100 °C for 30 minutes, at between 3.5 and 4.0 MPa.

Glass fibre rovings impregnated with polyester resin were wound around the stem of the prosthetic device and cured to produce a sheath about 5 mm thick, simulating the mechanical effect of the cortical bone surrounding the prosthetic device in the femur. Fatigue simulation testing of the prosthetic device within the sheath under ambient conditions showed that it could withstand a certain physiological load for over 300,000 cycles without failure, ie suggesting that the prosthetic device according to the present invention has fatigue endurance approximately the same as that of commercially available metal hip joint replacements.

The aforementioned physiological load, regarded in the art as that typically generated during walking, was 3 kN applied at the centre of gravity of the head at a physiological angle, ie 17° between load application line and stem axis.

The aforedescribed preparative procedures, ie moulding and curing steps, can be repeated except that the rectangular head could be replaced with a head of acceptable anatomical shape, eg a sphere of appropriate size.

Example 5

This Example illustrates the preparation of a prosthetic device according to the process of the present invention.

The mechanical properties of the prosthetic device determined in Example 4 were fed into a computer loaded with a PAFEC model of a Sheehan type femoral replacement. From the data generated in Example 4, photoelastic stress analysis of the model used therein and by a process of re-iteration an improved shape and size of femoral replacement was obtained.

## Claims

1. A prosthetic device characterised in that it comprises both a first component which comprises a modulus

matched composite which has a factor of safety of more than about 2 and a second component.

2. A prosthetic device as claimed in Claim 1 wherein the factor of safety is about 3.

3. A prosthetic device as claimed in Claim 1 in the form of a femoral replacement.

4. A prosthetic device in the form of a femoral replacement characterised in that it comprises at least both a stem prepared from a modulus matched composite and a second component provided with a metallic articulate surface and wherein for an applied load of 3 kN the stresses within the medial and lateral sides of the prosthesis are not more than about 50 MPa compressive and about 20 MPa tensile respectively.

5. A prosthetic device as claimed in Claim 3 or 4 wherein the second component provides the head of the femoral replacement.

6. A prosthetic device as claimed in Claim 5 wherein the head is metallic.

7. A prosthetic device as claimed in Claim 6 wherein the head is provided with a neck and a projection therefrom which is disposed within the proximal end of the composite stem.

8. A prosthetic device as claimed in Claim 7 wherein the neck is provided with a collar which provides direct compressive contact between the device and the bone adjacent the device.

9. A prosthetic device as claimed in Claim 1 or 4 wherein the composite component provides the major volume % of the prosthetic device.

10. A prosthetic device as claimed in Claim 9 wherein the composite component comprises 20-80% particulate inorganic solid by volume and wherein the cured resin in the composite component has a Young's modulus of about 3 GPa.

11. A prosthetic device as claimed in Claim 5 wherein the stem is provided with an outer layer of composite which contains less filler.

12. A process for the preparation of a prosthetic device which process comprises the steps of

A Estimating the target properties in a prosthetic device of known shape and size;

B Computing the stresses therein and in the bone adjacent thereto using FEA and photoelastic stress analysis;

C Redesigning a prosthetic device from the data generated in Steps A and B to produce physiological level of stresses in the said bone;

D Choosing a composite having the target properties and a suitable FOS for that application from the stress analysis produced in Step B and the mode of fatigue failure;

E Curing a curable composition to produce the composite chosen in Step D in a suitable mould for the redesigned prosthetic device from Step C;

F Tasting the redesigned prosthetic device from Step E in full-scale laboratory simulations while observing the time and mechanism of failure;

G Further redesigning the prosthesis from the data generated in Step F and optionally choosing a new composite to improve both FOS and stress transfer from the prosthesis to the adjacent bone; and

H Iterating the above Steps.

13. A process for the preparation of a prosthetic device as claimed in Claim 1 or 4 comprising the steps of disposing the second component in an appropriate mould, charging a curable composition to the mould and curing the curable composition to form the first component.

14. A process as claimed in Claim 13 wherein the first component provides the stem of a femoral device and the second component provides at least the head of a femoral device.

Fig 1

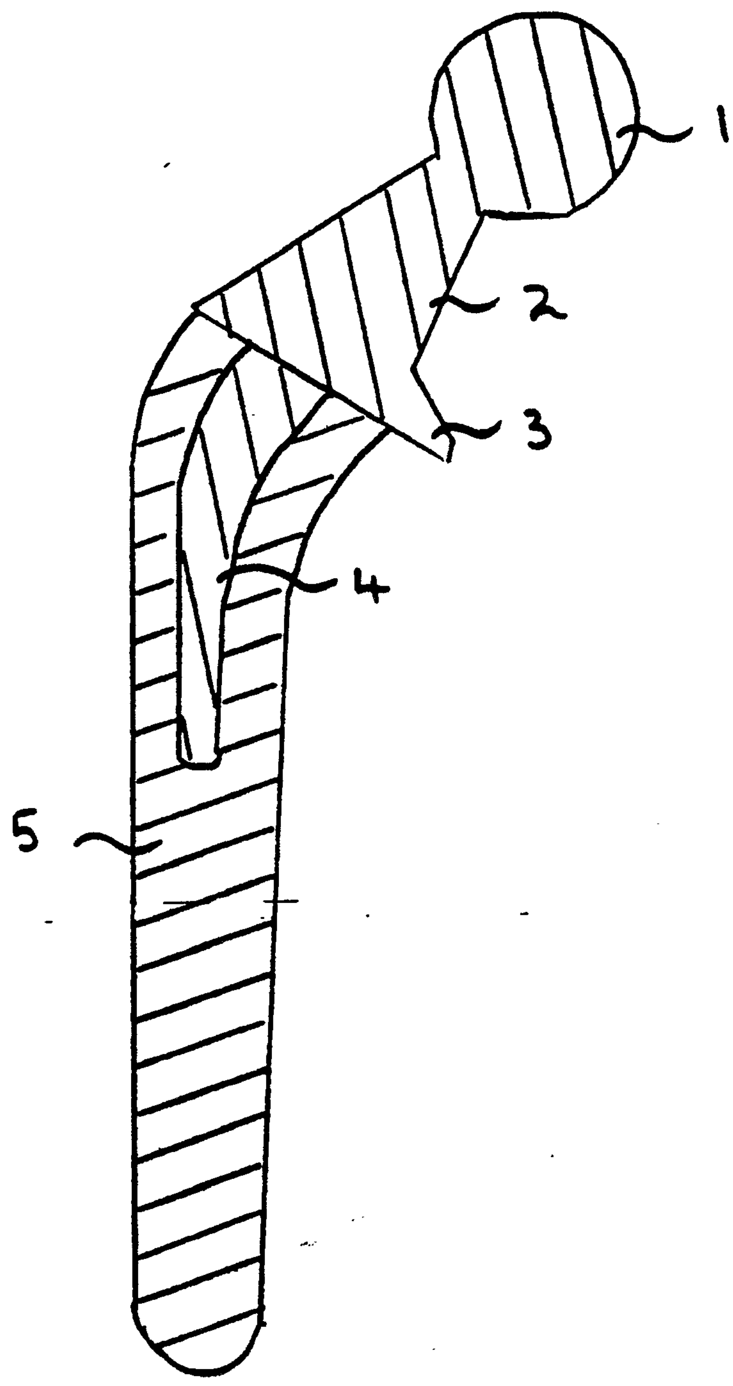

Fig 2

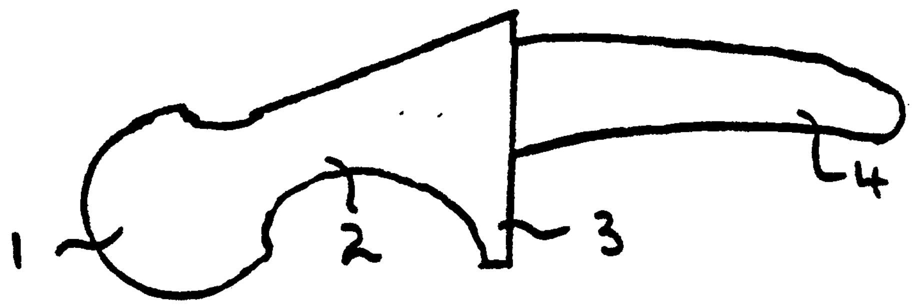

Figure 3

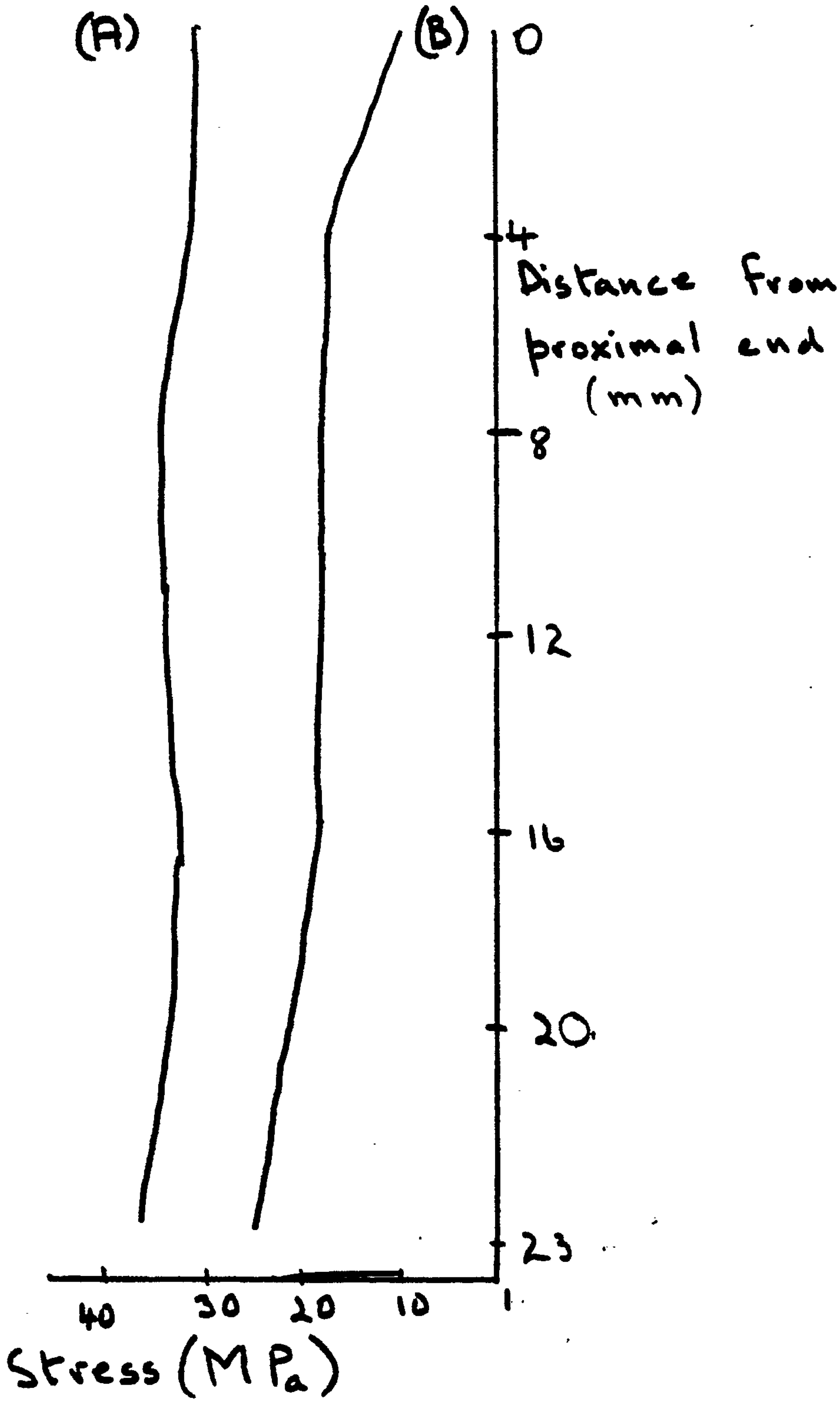
(A)
(B)
0
4
8
12
16
20
23
Distance from proximal end (mm)
40
30
20
10
Stress (MPa)

Figure 4